# EUROPEAN PATENT SPECIFICATION

(11) **EP 2 863 751 B1**
(45) Date of publication and mention of the grant of the patent: **25.07.2018**
(21) Application number: 13735469.2
(22) Date of filing: 08.05.2013
(51) Int. Cl.: A01P 1/00, A01N 25/08, A01N 59/16, A61K 33/24, B82B 3/00, B82Y 30/00, A61K 47/69

(54) **FUNCTIONALIZED HYDROXYAPATITE/GOLD COMPOSITES AS "GREEN" MATERIALS WITH ANTIBACTERIAL ACTIVITY AND THE PROCESS FOR PREPARING AND USE THEREOF**
FUNKTIONALISIERTER HYDROXYAPATIT/GOLD VERBUNSTOFF ALS "GRÜNES" MATERIAL MIT ANTIBAKTERIELLER AKTIVITÄT
COMPOSITE HYDROXYAPATITE/OR FONCTIONALISÉ EN TANT QUE MATERIAU "VERT" ANTIBACTERIEN

(30) Priority: 15.06.2012 SI 201200204
(43) Date of publication of application: 29.04.2015
(73) Proprietor: Institut "Jozef Stefan", 1000 Ljubljana (SI)
(72) Inventor: VUKOMANOVIC, Marija, Jagodina (RS); SKAPIN, Sreco Davor, SI-1358 Log (SI); SUVOROV, Danilo, SI-1000 Ljubljana (SI)
(74) Representative: Grünecker Patent- und Rechtsanwälte PartG mbB
(86) International application number: PCT/SI2013/000025
(87) International publication number: WO 2013/187846

(56) References cited:
- JP-A- 2010 082 776
- RYAN D. ROSS ET AL: "Binding affinity of surface functionalized gold nanoparticles to hydroxyapatite", JOURNAL OF BIOMEDICAL MATERIALS RESEARCH PART A, vol. 99A, no. 1, 1 October 2011 (2011-10-01), pages 58-66, XP055059995, ISSN: 1549-3296, DOI: 10.1002/jbm.a.33165
- YUYUN ZHAO ET AL: "Small Molecule-Capped Gold Nanoparticles as Potent Antibacterial Agents That Target Gram-Negative Bacteria", JOURNAL OF THE AMERICAN CHEMICAL SOCIETY, vol. 132, no. 35, 8 September 2010 (2010-09-08), pages 12349-12356, XP055081089, ISSN: 0002-7863, DOI: 10.1021/ja1028843 cited in the application
- JAMEE BRESEE ET AL: "Growth Inhibition of Staphylococcus aureus by Mixed Monolayer Gold Nanoparticles", SMALL, vol. 7, no. 14, 19 July 2011 (2011-07-19), pages 2027-2031, XP055081112,
- WEI CHEN ET AL: "Sonochemical Processes and Formation of Gold Nanoparticles within Pores of Mesoporous Silica", JOURNAL OF COLLOID AND INTERFACE SCIENCE, vol. 238, no. 2, 1 June 2001 (2001-06-01), pages 291-295, XP055081120, ISSN: 0021-9797, DOI: 10.1006/jcis.2001.7525
- OKITSU ET AL: "Sonochemical synthesis of gold nanoparticles on chitosan", MATERIALS LETTERS, NORTH HOLLAND PUBLISHING COMPANY. AMSTERDAM, NL, vol. 61, no. 16, 1 June 2007 (2007-06-01), pages 3429-3431, XP022079111, ISSN: 0167-577X, DOI: 10.1016/J.MATLET.2006.11.090
- DEBABRATA RAUTARAY ET AL: "Synthesis of Hydroxyapatite Crystals Using Amino Acid-Capped Gold Nanoparticles as a Scaffold", LANGMUIR, vol. 21, no. 11, 1 May 2005 (2005-05-01), pages 5185-5191, XP055081129, ISSN: 0743-7463, DOI: 10.1021/la048541f cited in the application
- NIRMALA R ET AL: "Bactericidal Activity and In Vitro Cytotoxicity Assessment of Hydroxyapatite Containing Gold Nanoparticles", JOURNAL OF BIOMEDICAL NANOTECHNOLOGY, AMERICAN SCIENTIFIC PUBLISHERS, US, vol. 7, no. 3, 1 January 2011 (2011-01-01) , pages 342-350, XP009172957, ISSN: 1550-7033, DOI: HTTP://DX.DOI.ORG/10.1166/JBN.2011.1292
- MARCOS DÍAZ ET AL: "Synthesis and Antimicrobial Activity of a Silver-Hydroxyapatite Nanocomposite", JOURNAL OF NANOMATERIALS, vol. 28, no. 2, 1 January 2009 (2009-01-01), pages 157-6, XP055081321, ISSN: 1687-4110, DOI: 10.1021/cr030027b

## Description

### Technical field of invention

Invention belongs to the technical field of construction of materials with antibacterial activity, in particular to materials formed of ceramic, metallic and functionalizational organic components whose combination result in formation of materials applicable against bacteria. The materials are prepared using sonochemical approach.

### Description of the technical problem

Technical problem, resolved by this invention, is a selection of components and their appropriate combination in order to form materials with adequate level of efficacy of antibacterial activity and toxicity. Materials made to be applied for protection against development of bacteria should have: (i) wide range of antibacterial activity, including activity against both, Gram positive and Gram negative bacteria and (ii) high efficiency of antibacterial activity in selected bacterial range. Together with a high efficacy against broad spectrum of bacteria these materials should be: (i) nontoxic for human beings and their environment and (ii) prepared using "green" technology. The currently used materials have antibacterial action along with a distinct level of toxcisity. For these material higher effectiveness against bacteria is marked by increased toxcisity to human.

### Proposed solution to the technical problem

Mentioned technical problem is resolved by the new process of preparing the material, as defined in claims, said material being constructed by the following selection of components: (i) hydroxyapatite (HAp), (ii) gold (Au) nanoparticles attached onto the surface of ceramic and (iii) organic molecules as defined in claims as functionalizations bonded on metallic surface. Metallic nanoparticles with functionalized surface are center of antibacterial activity while ceramics has a role of a templating agent. So constructed material has the following properties: (i) wide range of antibacterial activity against both Gram positive and Gram negative bacteria, (ii) very high efficiency of antibacterial activity in selected antibacterial region, (iii) non-toxicity for human and environment and (iv) possibility to be prepared by "green" approach without application of toxic precursors.

### Background of the invention

Mutations of bacteria as a result of stress induced by exposure to antibacterial agents cause formation of new strains of bacteria with a high ability for resistance and appearance of the multi-resistivity. This caused a need for a continuous development of new classes of antibiotics or for a constant need for increasing the range of their antibacterial activity. An alternative to the use of antibiotics for some specific usages, like prevention of development of infections, is to use inorganic sources able to have antibacterial activity. Many different metals with natural ability to have antibacterial activity (like zinc, copper, silver, titanium, etc.), their compounds or composites with other materials (ceramics, polymers and/or glasses) have potential to be applied for this purpose (EP1329211A1). So far these materials have been investigated in the form of bulk, micro-/nanoparticles or ions. As an example it has been shown that incorporation of Ti-ions within apatite structure provides possibility for formation of material applicable for formation of antibacterial paints (EP1985675A1) while deposition of HAp/ZnO nanoparticles onto the surface of Ti-implants allows formation of material able to promote osseointegration and to provide antibacterial properties (WO2011/022642A1).

However, so far silver has been considered as the most effective inorganic antibacterial agent. This metal is a natural antibiotic with a wide range of antibacterial activity including Gram positive and Gram negative bacteria. Moreover, except bacteria this metal is effective against many other pathogens including viruses and fungi. Specific mechanism of its action (related to destruction of cellular membranes) prevents failure of its activity and there is no pathogen with developed resistance to this metal. Because of all these benefits this metal has been widely used in practice and there is a enormous number of products available in the market containing silver, including cosmetics, medical devices, medicines, clothing, paintings, filters, air conditions, appliances, etc. As antibacterial agent it has been formulated in the following forms: hydrosol (WO 2010/143075 A2); metallic layer (EP2229962A1), metallic nanoparticles (WO2010/139451A2) or metallic ions (EP1819372A1) made onto/in different titanium-based, ceramic or glass/ceramic implants; metal oxide within bioactive glass (WO00/76486A1); metallic nanoparticles within polymeric support (WO2008/122131A1); etc. Composites of this metal with bioceramics (as hydroxyapatite) have been made by supporting of metallic nanoparticles on the surface of ceramics (US2010/0331574A1) or by simultaneous incorporation of metallic particles inside and their deposition on the surface of ceramics (WO2010/122354A1). So obtained materials were highly efficient in catalysis and applicable for formation of implants in orthopedics and dentistry.

Other members of noble metals (gold and platinum) are good candidates for silver replacement. These materials are inert which makes them "green" and nontoxic for human and its environment. However these materials do not have natural ability for antibacterial activity and in their case this property needs to be designed. It has been shown that gold has a possibility to be capped by different organic molecules containing amino or thiol groups. It is a well-known strategy for formation of stable and dispersive nanoparticles. So obtained nanoparticles, with addition of magnetic component, have been used for targeting of antibiotics to the bacteria in order to increase their activity (WO2007/015105A2). Moreover, it has been also observed that gold has a possibility to enhance and extend effects of pharmaceuticals after they have been attached on Au nanoparticles. Some examples of increased antibacterial activity of natural antibiotics bonded to Au nanoparticles are Au stabilized by gallic acid (S. A. Moreno-Alvarez et al.), ibuprofen-Au (A. T. M. Fiori et al.) and vancomycin-Au (H. Gu et al.). There are also examples for attachment of synthetic amino-substituted pyrimidines onto Au nanoparticles able to provide activity against Gram negative bacteria (Y. Zhao et al.) as well as formation of Au/carbon core-shell structure (Y.-H. Gao et al.) which efficiently succeeded in development of antibacterial activity.

There is also a library of 120 Au-nanoparticle conjugates with a combination of different natural antimicrobials simultaneously bonded onto the surface of Au nanoparticles (using thiol exchange reaction). The antibacterial activity of these conjugates is dependent on the combination of conjugating molecules simultaneously bonded to nanoparticles and is enhanced after bonding (J. Bresee et al.)

As it was shown by the firstly invented, natural-sourced antibiotic, the nature provides very good models for designing materials for the specific applications such as antibacterial activity. Prior art shows that starting from this idea, Au nanoparticles may be synthesized using biosynthetic process where natural sources (plants, bacteria, honey or propolis) were used. This approach is able to provide antibacterial activity of resultant material. Efficiency of the action of these materials against bacteria was low however they provided a good direction for possibility for the application of natural sources for designing antibacterial property of this metal (D. MurbarakAli et al.). There is a possibility for formation of the complex between Au-ions and amino acids (glycine, histidine and tryptophan) and it has been shown that among formed complexes, Au-glycine and Au-tryptophan were those who had antimicrobial activity (A. S. Kazachenko et al.). So far different amino acids have been attached onto the surface of gold nanoparticles. Some of them were used for stabilization of the gold surfaces as capping agents. These agents (like glutamic acid and histidine, (L. Polavarapu et al.) or alanine and arginine (M. Koyama et.al.)) were attached on the surface of metallic nanoparticles after they have been formed using other reduction agents. Some of these amino acids were used for reduction of gold (aspartic acid,(S. Mandal et al.), tryptophan, (P. R. Selvakannan et al.), L-aspartat,(Y. Shao et al.) as well as tyrosine, glycil-l-tyrosine and L-arginine (S. K. Bhargava et al.)) and this reaction was performed under increased temperature or pH of the system. For the case of preparation of catalyst, gold nanoparticles were supported onto the surface of previously synthesized hydroxyapatite by the procedure of deposition-precipitation. So obtained material was able to induce catalytic activity for CO oxidation (M. I. Dominquez et al.). In the case of formation of material which will be useful as a biosensor this material has been formulated in a different ways. One of the examples is formation of material able to be used for protein adsorption analysis and in that case thin film of hydroxyapatite nanocrystals were deposited onto the surface of gold substrate (A. Monkawa et al.). Another example concerns formation of biosensor for electrocatalytic detection of hydrogen peroxide and for that purpose hemoglobin was immobilized onto the gold-hydroxyapatite composite. This composite was formed of hydroxyapatite nano tubes with deposited gold nanoparticles onto their surface (J. You et al.). Additional formulation concerns gold which was capped by natural proteins, collagen (S. Aryal et al.) or fibrin (T. P. Sastry et al.), as well as by the nucleobase polymer (Y. Gupta et al.) and their deposition onto hydroxyapatite.

Similarly, pre-formed gold nanoparticles (formed by citrate reduction and functionalized with L glutamic acid, 2-aminoethylphosphonic acid or alendronate) were shown to have ability to be adsorbed onto bone mineral (using hydroxyapatite as a model) which highlighted their applicability as contrast agent for labelling damaged bone tissue and detection of calcification in soft tissue associated with cancer or musculoskeletal injury and as drug delivery vehicle (R. D. Ross et at.).

Hydroxyapatite and gold have also been considered as components of coatings for production of biocompatible surfaces. These coatings are formed by using depositing techniques (lithographic pattering or thin film deposition) and bonded to the biocompatible surface by compression, electric arc spot welding or high temperature diffusion bonding method. The main purpose of formation of these coatings is in formation of bioactive surface layer. They will be used in order to improve the interaction of product with cells which is important for different devices that will contain this product (implants, biochips, bioreactors, biosensors, etc.) Among numerous other purposes so obtained surfaces are used for production of cell substrates and they could contain bioactive agent within multi-functional implant device. This device comprises bioactive agents from the group of pharmaceuticals, therapeutic drugs, cancer drug, growth factor, protein, enzyme, hormone, nucleic acid, antibiotic, nanoparticle and biologically active material. The device is able to provide controlled release of the active agent by the application of external (electromagnetic or ultrasonic) field.(WO 2008/066965)

Hydroxyapatite and gold have also been included within the method for regulation of cellular function. This method comprises combination of material and nanoparticle composition in the form of material system. Nanoparticle composition has core-shell structure and it coats surface of material. These core/shells are 50 µm to 50 mm in size and can be made of various materials including gold, metal, metal oxide, polymer, titanium dioxide, silver, carbon nanotubes, hydroxyapatite, quantum dots, crystals, salts, ceramic materials, magnetic materials and any combination thereof. It has been stated that mentioned material system is capable of preventing or treating infection, however it has not been specified exactly which combinations of these materials is able to provide anti-infective effect since all of them are not able to do so. Material system has been described as a surface coating or as a system for delivery of the drugs and bioactive substances. In the first case when material is used as a coatings on the surface of medical devices and implants it has been précised that gold or hydroxyapatite are used for the promotion of the tissue growth. If drug delivery is concerned, these systems contain at least one agent (protein, growth factor, antibody, amino acid, polymer, drug, hormone, nucleic acid, peptide, or enzyme) linked to the nanoparticle. It has been specified that the cellular function that can be regulated by so developed stimulating agent made of nanoparticle linked with mentioned agent(s) is bone growth.(WO2010/062561)

### Disadvantages of currently available technical solutions

Despite the fact that silver has very strong and nonselective activity against many different pathogens, its inability to make a difference between cells of pathogens and other live cells is a serious lack of this metal when its antibacterial activity is considered. This property makes it toxic for human beings and their environment, which is the main reason for the urgent need to replace silver with a "green" and human friendly substitute, with at least the same properties as silver with respect to antibacterial activity.

Other noble metals, such as gold nanoparticles, are nontoxic. However they do not posess natural antibacterial activity and in their case this property needs to be designed, e.g. by a complexation of gold with some amino acids, which then allows for antibacterial activity (A. S. Kazachenko et al.). However, so far amino acids were used either for reduction or for stabilization of the surface of already formed gold nanoparticles (L. Polavarapu et al.; M. Koyama et al.; S. Mandal et al.; P. R. Selvakannan et al.; Y. Shao et al.; S. K. Bhargava et al.) and resulted materials have not been considered as materials with antibacterial activity.

On the other hand, hydroxyapatite is a well known bioceramics which is a major content of inorganic part of natural bones. This material has very high bioactivity and possibility to stimulate formation of cells and reparation of tissue and therefore a high potential to contribute to the better biocompatibility and bioactivity of antibacterial material, as its support, as already presented in the case of silver/hydroxypatite composites (US2010/0331574A1; WO2010/122354A1).

Because of their advantages hydroxapatite/gold composites have been considered for different biomedical and non-biomedical applications such as: (1) for catalytic activity in oxidation of CO (M. I. Dominguez et al.), when gold nanoparticles were deposited on already formed apatite; (2) for electrocatalytic detection of hydrogen-peroxide as biosensor with and without hemoglobin deposited onto the surface of gold-hydroxyapatite (A. Monkawa et al.; J. You et al.) or by capping gold by natural polymers and their deposition onto hydroxyapatite (S. Aryal et al.; T. P. Sastry et al.; Y. Gupta et al.); (3) detection of protein adsorption as biosensor (A. Monkawa et al.) when hydroxyapatite thin film was deposited onto the surface of gold substrate; and (4) regulation of the cell function by promoting of the cellular growth and as carriers of the drugs (including known anti-infective agents) in the form of bioactive coatings (WO2010/062561, WO 2008/066965).

Composites of hydroxyapatite with noble metals have been reported for designing antimicrobial agents. Examples are hydroxyapatite/gold with gelatin and chitosan used as binding agents for attachment of hydroxyapatite over gold nanoparticles (R. Nirmala et al.) as well as hydroxyapatite/silver nanocomposites formed of silver nanoparticles supported onto the surface of hydroxyapatite (M. Diaz et al.). In both examples natural antimicrobial agents (chitosan or silver) were applied to design antibacterial activity in the developed composites.

There is a literature reporting on the possibility to form gold nanoparticles using sonochemical approach. Gold can be formed in pure organic solvents (ethanol, methanol and DMF) in the presence of organic stabilizers (carboxylic acid, amine, thiol and organophosphoric compound) (JP 2010 082776). In another study, formation of gold nanoparticles took place within silica mesopores using isopropanol-assisted sonochemical approach. The process lasts for 3 weeks in argon inert atmosphere by immersing the porous structures into reaction medium to obtain entrapment of gold nanoparticles inside pores (W. Chen et al.). Gold nanoparticles were also made during the process of sonication in inert argon atmosphere with addition of small quantity of isopropanol and dispersed solid chitosan when formed gold nanoparticles remined attached onto chitosan (K. Okitsu et al). Application of sonochemical method for the production of functionalized hydroxyapatite/gold composite material has not been concerned in the literature.

According to the literature, antibacterial material formed of hydroxiaptite arid gold in the form of composite with the surface functionalized by amino acids las described in the appended claims has not been reported.

Procedures developed for formation of HAp/Au are different variations of deposition procedure (deposition of HAp onto previously synthesized gold/protein matrix or deposition of Au on previously formed HAp). This approach usually results in particles agglomeration and low stability of Au nanoparticles attached onto HAp or vice versa.

Application of sonochemical method for the production of this type of material has not been concerned in the literature.

Summary of cited patents
- T. Saito, H. Nakanaga, Antibacterial resin, EP1329211A1, 2003.
- M. Wakamura, K. Masato, Antibacterial paint for materials and materials coated therewith, EP1985675A1, 2008.
- P. A. Malshe, W. Jiang, Nanostructured hydroxyapatite coatings for dental and orthopedic implants, WO2011/022642A1, 2011.
- Willoughby, Dental uses of silver hydrosol, WO 2010/143075 A2, 2010.
- L. Martelli, N. Tottolo, M. Franco, M. Martelli, Medical-surgical devices with antibacterial coating for human or animal implant and a method for their production, EP2229962A1, 2010.
- E. Dingeldein, C. Gasqueres, F. Witte, A. Elizier, Osteosynthesis with nano-silver, WO2010/139451A2, 2010.
- S. Di Nunzio, E. Verne, Process for the production of silver-containing prosthetic devices, EP1819372A1, 2007.
   A. Masafumi, Nano-particle synthetic method by organic solvent sono-chemistry, JP 2010 082776 A (Univ.Tohoku), 2010.
- M. Bellantone, J. N. Coleman, L. L. Hench, Silver-containing, sol-gel derived bioglass compositions, WO 00/76486A1, 2000.
- S. Bokorny, J. W. Stark, F. S. Loher, Antimicrobial material, WO 2008/122131 A1, 2008.
- K. Kiyotomi, Y. Noritsugu, Hydroxyapatite with the silver supported on the surface thereof, US 2010/0331574A1, 2010.
- H. E. Lester, Hydroxyapatite material and methods of production, WO 2010/122354 A1, 2010.
- W. T. Rademacher, G. Bradman, S. Penades Ullate, R. Ojeda Martinez De Castilla, Nanoparticles comprising antibacterial ligands, WO 2007/015105 A2, 2007.
- S. Jin, S. Oh, Articles comprising large-surface-area bio-compatible materials and methods for making and using them, WO 2008/066965 A2, 2008.
- A. S. Biris, M. Mahmood, P. Jensen, Advanced nanomaterials for regulating specific cell functions, WO 2010/062561 A1, 2010.

### Summary of cited non-patent sources

- S. A. Moreno-Alvarez, G. A. Martinez-Castanon, N. Nino-Martinez, J. F. Reyes-Macias, N. Patino-Marin, J. P. Loyola-Rodriquez, F. Ruiz, Preparation and bactericide activity of gallic acid stabilized gold nanoparticles, J. Nanopart. Res. 12 (2010) 2741-2746.
- T. M. Fiori, W. R. Lustri, A. Magalhaes, P. P. Corbi, Chemical, spectroscopic characterization and antibacterial activities of in vitro of a novel gold(l)-ibuprofen complex, Inorgan. Chem. Commun., 14 (2011) 738-740.
- H. Gu, P. L. Ho, E. Tong, L. Wang, B. Xu, Presenting vancomycin on nanoparticles to enhance antimicrobial activities, Nano Lett., 3 (2009) 1261-1263.
- Y. Zhao, Y. Tian, Y. Cui, W. Liu, W. Ma, X. Jiang, Small molecule-capped gold nanoparticles as potent antibacterial agents that target gram-negative bacteria, J. Am. Chem. Soc., 132 (2010) 12349-12356.
- Y.-H. Gao, N.-C. Zhang, Y.-W. Zhong, H.-H. Cai, Y.-L. Liu, Preparation and characterization of antibacterial Au/C core-shell composite, Appl. Surf. Sci., 256 (2010) 6580-6585.
- D. MubarakAli, N. Thajuddin, K. Jeganathan, M. Gunasekaran, Plant extract mediated synthesis of silver and gold nanoparticles and its antibacterial activity against isolated pathogens, Colloid. Surf. B, 85 (2011) 360-365.
- A. S. Kazachenko, E. V. Legler, O. V. Peryanova, Y. A. Vstavskaya, Synthesis and antimicrobial activity of gold complexes with glycine, histidine, and tryptophan, Pharm. Chem. J., 33 (1999) 470-472.
- L. Polavarapu, Q.-H. Xu, A single-step synthesis of gold nanochaines using an amino acid as a capping agent and characterization of their optical properties, Nanotechnol. 19 (2008) 075601 (6pp).
- M. Koyama, M. Shirashi, K. Sasaki, K. Kon-no, Preparation of L-alaninine crystals containing gold nanoparticles, J. Disper. Sci. Technol., 29 (2008) 1266-1271.
- S. Mandal, P. R. Selvakanrian, S. Phadtare, R. Pasricha, M. Sastry, Synthesis of stable gold hydrosol by the reduction of chloroaurate ions by the amino acid, aspartic acid, Proc. Indian Acad. Sci., (Chem. Sci.), 114 (2002) 513-520.
- P.R. Selvakannan, S. Mandal, S. Phadtare, A. Gole, R. Pasricha, S. D. Adyanthaya, M. Sastry, Water-dispersible tryptophan-protected gold nanoparticles prepared by the spontaneous reduction of aqueous chloroaurate ions by the amino acid, J. Colloid. Interf. Sci., 269 (2004) 97-102.
- Y. Shao, Y. Jin, S. Dong, Synthesis of gold nanoplates by aspartate reduction of gold chloride, Chem. Commun., 7 (2004) 1104-1105.
- S. K. Bhargava, J. M. Booth, S. Agrawal, P. Coloe, G. Kar, Gold nanoparticles formation during bromoaurate reduction by amino acids, Langmuir, 21 (2005) 5949-5956.
- M. I. Dominguiez, F. Romero-Sarria, M. A. Centeno, J. A. Odriozola, Gold/hydroxypatite catalysts synthesis, characterization and catalytic activity, Appl. Catal. B-Environ., 87 (2009) 245-251.
- A. Monkawa, T. Ikoma, S. Yunoki, T. Yoshioka, J. Tanaka, D. Chakarov, B. Kasemo, Fabrication of hydroxypatite ultra-thin layer on gold surface and its application for quartz crystal microbalance technique, Biomaterials, 27 (2006) 5748-5754.
- J. You, W. Ding, S. Ding, H. Ju, Direct electrochemistry of hemoglobin immobilized on colloidal gold-hydroxyapatite nanociomposite for electrocatalytic detection of hydrogen peroxide, Electroanal., 21 (2009) 190-195.
- S. Aryal, R. K. C. Bahadur, S. R. Bhattarai, P. Prabu, H. Y. Kim, Immobilization of collagen on gold nanoparticles: preparation, characterization, and hydroxyapatite growth, J. Mater. Chem., 16 (2006) 4642-4648.
- T. P. Sastry, J. Sundaraseelan, K. Swarnalatha, S. S. Liji Sobhana, M. Uma Makheswari, S. Sekar, A. B. Mandal, Growth of hydroxyapatite on physiologically clotted capped gold nanoparticles, Nanotechnol., 19 (2008) 245604 (5pp).
- Y. Gupta, G. N. Mathur, S. Verma, Biomimetic synthesis and Ultrastructural characterization of a zerovalent gold-hydroxyapatite composite, Bioorgan. Med. Chem. Lett., 16 (2006) 363-366.
- R. D. Ross, R. K. Roeder, Binding affinity of surface functionalized gold nanoparticles to hydroxyapatite, J. Biomed. Mater. Res. A., 99 (2011) 58-66.
- J. Bresee, K. E. Maier, A. E. Boncella, C. Melander, D. L. Feldheim, Growth Inhibition of Staphylococcus aureus by Mixed Monolayer Gold Nanoparticles, Small, 7 (2011) 2027-2031.W. Chen, W, Cai, L. Zhang, G. Wang, L. Zhang, Sonochemical process and formation of gold nanoparticles within pores of mesoporous silica, J. Colloid Interf. Sci., 238 (2001) 291-295.
- K. Okitsu, Y. Mizukoshi, T. A. Yamamoto, Y. Maeda, Y. Nagata, Sonochemical synthesis of gold nanoparticles on chitosan, Mater. Lett., 61 (2007) 3429-3431.
- R. Nirmala H. M. Park, D. Kalpana H. S. Kang, R. Navamathavan, Y. S. Lee, H. Y. Kim, Bactericidal activity and in vitro cytotoxicity assessment of hydroxyapatite containing gold nanoparticles, J. Biomed. Nanotechnol. 7 (2011) 342-350.
- M. Diaz, F. Barba, M. Miranda, F. Guitián, R. Torrecillas, J. S. Moya, Synthesis and antimicrobial activity of a silver-hydroxyapatite nanocomposite, J. Nanomater., 2009 (2009) 498505.
- D. Rautaray, S. Mandal, M. Sastry, Synthesis of hydrxyapatite crystals using amino acid-capped gold nanoparticles as a scaffold, Langmuir, 21 (2005) 5185-5191.

### Summary of the invention

Currently available technologies are using toxic silver as the most efficient inorganic antibacterial material. At the same time, some other materials which are nontoxic and have possibility to expose activity against bacteria, are not considered for this purpose.

Starting from these points of view, it is the object of the presented invention to provide a novel solution for a design of material made of non-toxic, natural or non-natural sourced components with strong antibacterial activity able to be used as "green" and effective replacement of silver, as well as the procedure for producing the same.

This invention concerns the antibacterial composite prepared by sonochemical precipitation, the composite comprising: a) a hydroxyapatite substrate, b) gold nanoparticles, and c) organic molecules, wherein the gold nanoparticles are attached to the hydroxyapatite substrate surface; the gold nanoparticles are covered by the organic molecules; the organic molecules are selected from the group consisting of arginine, glycine, aniline, histidine, 4(Methylthio)aniline and 5-bromopyridine-2-thiol. The material was tested and confirmed to have antibacterial activity.

Invented material has been synthesized by the application of a novel sonochemical precipitation method. In this invention procedure the sonication of apatite structured material activated its surface and allowed strong attachment of Au nanoparticles based on electrostatic interactions.

### Detailed description of the invention

This invention is concerned with:
1. Material: Hydroxyapatite/gold composite with the surface functionalized by organic molecules as defined in claims, as a novel concept for selection of its building components able to provide activity against bacteria and nontoxcisity.
2. Synthesis procedure: A novel procedure to the synthesis of functionalized hydroxyapatite/gold composite as defined in claims.
Innovative solutions for formation of antibacterial material presented in this patent are going to be described according to the Figures which present:

### Description of drawings

- Figure 1:: Schematic presentation of the new concept for construction of material with antibacterial activity formed of hydroxyapatite (1), gold (2) and organic functionalizational components with amine (3) and/or thiol groups (4).
- Figure 2:: Example of the morphological properties of invented materials: functionalized gold nanoparticles (gold/arginine) (1) and composite formed of HAp particles as templates (2) for nanosized gold particles (3,4) with functionalized surfaces (hydroxyapatite/gold/arginine).
- Figure 3:: Example of the absence of antibacterial activity of the separated components (pure functionalizations- glycine (1,10), arginine (2,11) and histidine (3,12) amino acids and HAp/functionalizations- HAp/glycine (4,13), HAp/arguinine (5,14) and HAp/histidine (6,15)) of against the same Gram positive and Gram negative bacteria, respectively, and intensive antibacterial activity of HAp/Au materials with three different functionalizations (HAp/Au/glycine (7,16), HAp/Au/arginine (8,17) and HAp/Au/histidine (9,18)) against the same type of bacteria.
- Figure 4:: Example for the absence of antibacterial activity of HAp (1,4) and lower effectiveness of antibacterial activity of HAp/Ag composite (2,5) compare to activity of the ceramic/metallic composite with functionalized surface (HAp/Au/arginine amino acid) (3,6) against Gram positive and Gram negative.

### Detailed Description of invention

### 1. MATERIAL

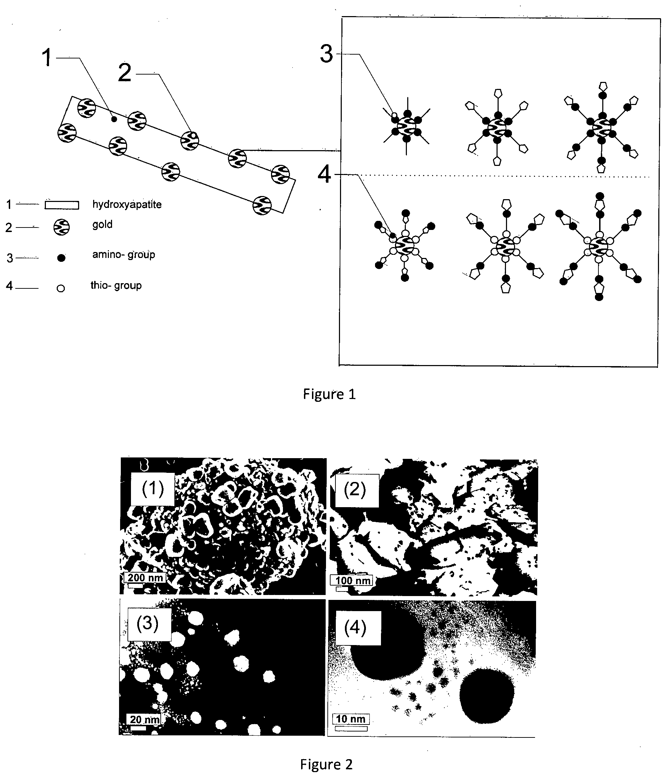

Developed material is a powder. The material is a composite and its structure is formed of two building components (illustrated in Figure 1):
(i) Bioceramic particles which are carriers of the component with antibacterial activity, characterized by being hydroxyapatite rods (Figure 1: 1)
(ii) Metallic nanoparticles with functionalized surface which are centers of antibacterial activity, characterized by being Au nanoparticles (Figure 1: 2) with arginine, glycine, aniline, histidine (Figure 1: 3), and/or 4(Methylthio)aniline and 5-bromopyridine-2-thiol (Figure 1: 4).
   (i) Bioceramic particles are characterized by being:
      (1) Formed of hydroxyapatite phase composied of submicrometer-sized rod-like bioceramic particles; which are carriers of metallic component,
      (2) Prevention of aggregation and ingrowth of metallic component into larger structures which is characterized by existence of electrostatic interactions between bioceramic rods and metallic nanospheres attached on their surface.
   (ii) Metallic nanoparticles with functionalized surface are characterized by being:
      (1) Formed of gold as a metallic phase and different organic molecules attached onto gold surface.
      (2) Organic molecules attached onto gold surface (functionalizations) are characterized so that they are selected from the group consisting of arginine, glycine, aniline, histidine, 4(Methylthio)aniline and 5-bromopyridine-2-thiol.

### 2. SYNTHESIS PROCEDURE

Another important novelty of this invention is a procedure developed for formation of material designed according to the mentioned, new design able to provide antibacterial activity. Hydroxyapatite is synthesized using homogeneous precipitation method and precipitate is redispersed in water solution using high intensity ultrasound. During this process, small amount of isopropanol has been added in order to increase sonication effect, allow surface stabilization and to induce formation of reactive radicals. In the next step, water solution of functionalizing agent has been added. Sonication time depends on the type of functionalizing agent. After sonication is finished, white water dispersion of apatite turns into pink, red or violet which is an indication for formation of apatite/gold composite containing metallic nanoparticles.

### Example 1

Example one of this invention demonstrates how morphological properties of metallic nanoparticles can be prevented from agglomeration and ingrowth into larger structures and be kept stable.

Without a presence of hydroxyapatite template, gold particles with the presence of functionalization (arginin amino acid) on their surface are highly agglomerated and with the broad size distribution which demonstrated their instability (Figure 2: 1).

Precipitation of hydroxyapatite followed its processing using high-intensity sonication activates its surface. Reduction of gold using selected functionalization is followed by strong attachment of these nanopaticles onto apatite and formation of stable composite. In the case when hydroxyapatite template is present, morphological properties of gold particles are changed resulting in formation of small nanometer sized sphere-like particles with significantly narrower size distribution ranging from a few to 20 nm (Figure 2: 3,4). These nanometer size metallic particles are attached onto the surface of rod-like particles of ceramic phase as demonstrated in Figure 2: 2.

### Example 2

Example two of this invention demonstrates how antibacterial activity of developed material has been achieved and how intensive it is.

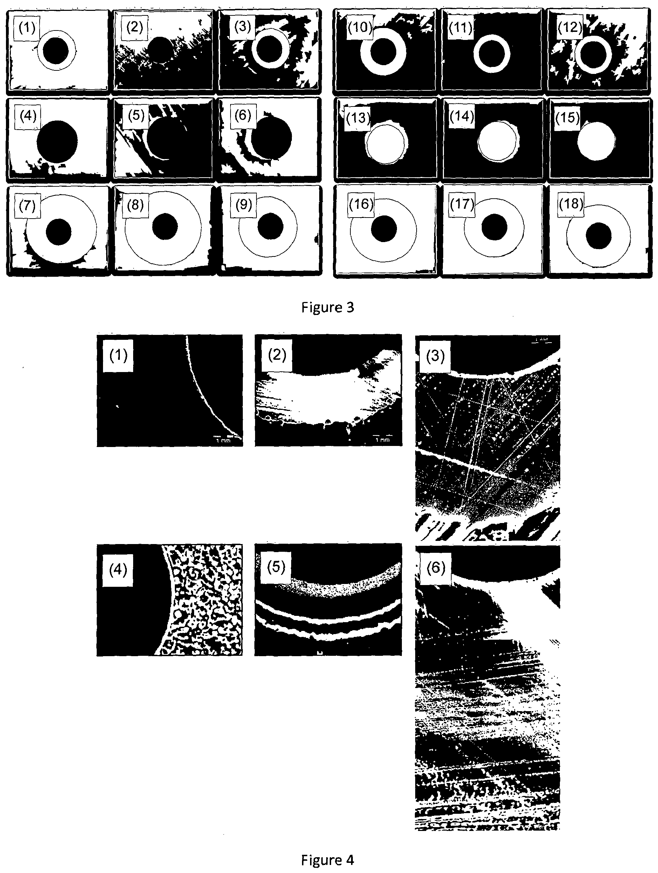

Organic molecules (glycin, arginine and histidine amino acids) used for functionalization of the surface of gold nanoparticles do not have or have very low antibacterial activity against Gram positive and Gram negative bacteria when metallic part is not present (Figure 3: 1-3, 10-12).

Hydroxyapatite, processed with the same organic materials (glycin, arginine and histidine amino acids) used for functionalization of gold surface according to this invention to form functionalized ceramics (HAp/ glycin, arginine or histidine amino acids), do not have antibacterial activity against Gram positive and Gram negative bacteria when metallic part is not present (Figure 3: 4-6, 13-15).

Antibacterial properties of material have been achieved by making a hydroxyapatite/gold composite and addition of selected functionalization to the surface of gold. This results in a different intensity of antibacterial action.

The strongest antibacterial activity is obtained for functionalization using natural organic molecules with the following order:
(i): arginine- with the strongest intensity of antibacterial activity (Fig. 3: 7,16)
(ii) glycine- with middle intensity of antibacterial activity (Fig. 3: 8,17)
(ii) histidine- with the weakest intensity of antibacterial activity (Fig. 3: 9,18)

Non-natural functionalizations have lower intensity of antibacterial activity compare to natural ones and the order of their activity against bacteria is:
(i) 5-bromopyridine-2-thiol- with the strongest intensity of antibacterial activity
(ii) 4(methilthio) aniline- with middle intensity of antibacterial activity
(iii) aniline- with the weakest intensity of antibacterial activity.

According to this invention combination of the surface functionalizations and gold nanoparticles are the main reason of antibacterial activity of invented hydroxyapatite/gold composite material with intensity which is the same or higher compare to apatite structured material/Ag (Figure 4: 1-6) for both Gram positive and Gram negative bacteria and which does not contain toxic components.

### Example 3

Example four of this invention concerns explanation of used procedure for the synthesis of hydroxyapatite/gold composite with functionalized surface of gold nanoparticles.

Process for preparing (or the synthesis procedure for formation) of mentioned hydroxyapatite/gold composite with functionalized surface of metal nanoparticles:
Hydroxyapatite (c=1.5 mg/ml) was dispersed into isopropanol solution in water (c=2·10⁻² ml/ml) using ultrasonication (time of sonication t=10 minutes, pulsation-to-relaxation periods on:off=02:01, T=25°C, power P=600W, frequency f=20 kHz and amplitude A=80%). After dispersion period was finished, 50 ml of aqueous solution of chloroauric acid (HAuCl4) (c=0.8 mg/ml) were added. This step was followed by addition of 50 ml of aqueous solution of selected functionalization (n(HAuCl4): n(functionalization) = 1 : 1 mol/mol). During the last two steps sonication was performed using the following parameters: pulsation-to-relaxation periods on:off=02:01, T=25°C, power P=600W, frequency f=20 kHz, amplitude A=80% and time of sonication that depended on selected functionalization. For amino acid sonication time was t=15 minutes, for aniline and its derivate t=5 minutes and for bromopyridine-2-thiol it was t=30 minutes. Obtained precipitates were separated from supernatant by centrifugation (15 minutes at 5000 rpm) and air-dried on glass slides.

### Industrial applicability of the invented material

Developed material has potential applications in followed fields:

### Antibacterial applications

- Filters (component of face-masks, respirators or antibacterial layer inside air-conditioners for elimination of bacteria from air in hospitals, laboratories and enclosed public and domestic places, antibacterial filters inside refrigerators, washing machines, vacuum cleaners, etc.)
- Food industry (component of chewing gums with antibacterial activity for prevention of caries and other dental and oral infections and as dietary supplement- Au is able to affect nervous system in order to handle stress while apatite structured bioceramics is a Ca-source.)
- Painting industry (as a component of dispersions for painting the walls- for domestic or public application; Ca-based bioceramic part has potential to provide better adhesion)
- Hygiene (component of toothpastes or mouthwash liquid for providing prevention of development of oral and dental infections as well as for improvement of mineral part of dental enamel; components of soaps for hand washing in public places such as public toilets)
- First-aid materials (antibacterial layer on adhesive plasters, bandages and gauze for wound treatment.)
- Dentistry (component of tooth (endodontic) fillers or/and material for formation of dental implants able to provide antibacterial activity and improvement of mechanical strength due to the presence of metallic part.)
- Cosmetics (deodorant, roll-on, shampoo, shower gel, etc.)

### Other applications

- Catalysis (for selective aerobic oxidation applicable for self-cleaning covers, water purification, pollutant)
- Therapy and diagnostics (formation of biodetectors for detection of biomineralization of implanted site and biosensor- for attachment and purification of biomacromolecules such as proteins and DNA)

## Claims

1. Antibacterial composite prepared by sonochemical precipitation, the composite comprising:
a) a hydroxyapatite substrate,
b) gold nanoparticles, and
c) organic molecules,
wherein the gold nanoparticles are attached to the hydroxyapatite substrate surface; the gold nanoparticles are covered by the organic molecules; the organic molecules are selected from the group consisting of arginine, glycine, aniline, histidine, 4(Methylthio)aniline and 5-bromopyridine-2-thiol.

2. Method for preparing an antibacterial composite comprising:
a) a hydroxyapatite substrate,
b) gold nanoparticles, and
c) organic molecules,
wherein the gold nanoparticles are attached to the hydroxyapatite substrate surface; the gold nanoparticles are covered by the organic molecules; the organic molecules are selected from the group consisting of arginine, glycine, aniline, histidine, 4(Methylthio)aniline and 5-bromopyridine-2-thiol; and the preparation is carried out by sonochemical precipitation.

3. Antibacterial composite according to claim 1 for use in therapy.

4. Antibacterial composite according to claim 1 for use in cosmetics.

5. A material comprising the antibacterial composite of claim 1, wherein the material is selected from the group consisting of a filter, a component of a face-mask, respirator, air-conditioner, refrigerator, washing machine or vacuum cleaner, a chewing gum, a dietary supplement, a dispersion for painting walls, a toothpaste, a mouthwash, a soap, a first-aid material, an adhesive plaster, a bandage, a gauze, a tooth filler, a material for forming dental implants, a deodorant, roll-on, shampoo, and shower gel.

## Patentansprüche

1. Antibakterielles Komposit, hergestellt durch sonochemische Präzipitation, das Komposit umfassend:
a) ein Hydroxylapatitsubstrat,
b) Goldnanopartikel, und
c) organische Moleküle,
wobei die Goldnanopartikel an die Oberfläche des Hydroxylapatitsubstrats gebunden sind; die Goldnanopartikel mit den organischen Moleküle überzogen sind; die organischen Moleküle ausgewählt sind aus der Gruppe bestehend aus Arginin, Glyzin, Anilin, Histidin, 4(Methylthio)anilin und 5-Bromopyridin-2-thiol.

2. Verfahren zur Herstellung eines antibakteriellen Komposits, umfassend:
a) ein Hydroxylapatitsubstrat,
b) Goldnanopartikel, und
c) organische Moleküle,
wobei die Goldnanopartikel an die Oberfläche des Hydroxylapatitsubstrats gebunden sind; die Goldnanopartikel mit den organischen Molekülen überzogen sind; die organischen Moleküle ausgewählt sind aus der Gruppe bestehend aus Arginin, Glyzin, Anilin, Histidin, 4(Methylthio)anilin und 5-Bromopyridin-2-thiol; und die Herstellung durch sonochemische Präzipitation durchgeführt wird.

3. Antibakterielles Komposit gemäß Anspruch 1 zur Verwendung in der Therapie.

4. Antibakterielles Komposit gemäß Anspruch 1 zur Verwendung in Kosmetika.

5. Ein Material umfassend das antibakterielle Komposit aus Anspruch 1, wobei das Material ausgewählt ist aus der Gruppe bestehend aus einem Filter, einer Komponente einer Gesichtsmaske, eines Respirators, einer Klimaanlage, eines Kühlschranks, einer Waschmaschine oder eines Staubsaugers, einem Kaukummi, einem Nahrungsmittelzusatz, einer Dispersion zum Streichen von Wänden, einer Zahnpasta, einer Mundspülung, einer Seife, eines Erste-Hilfe-Materials, einem Heftpflaster, einer Bandage, einer Gaze, einem Zahnfüllungsmittel, einem Material zum Fertigen von Zahnimplantaten, einem Deodorant, einem Deoroller einem Shampoo und einem Duschgel.

## Revendications

1. Composite antibactérien préparé par précipitation sonochimique, le composite comprenant :
a) un substrat d'hydroxyapatite,
b) des nanoparticules d'or, et
c) des molécules organiques,
les nanoparticules d'or étant fixées à la surface du substrat d'hydroxyapatite ; les nanoparticules d'or étant recouvertes des molécules organiques ; les molécules organiques étant sélectionnées dans le groupe constitué de l'arginine, de la glycine, de l'aniline, de l'histidine, de la 4-(méthylthio)aniline et du 5-bromopyridine-2-thiol.

2. Procédé de préparation d'un composite antibactérien comprenant :
a) un substrat d'hydroxyapatite,
b) des nanoparticules d'or, et
c) des molécules organiques,
les nanoparticules d'or étant fixées à la surface du substrat d'hydroxyapatite ; les nanoparticules d'or étant recouvertes des molécules organiques ; les molécules organiques étant sélectionnées dans le groupe constitué de l'arginine, de la glycine, de l'aniline, de l'histidine, de la 4-(méthylthio)aniline et du 5-bromopyridine-2-thiol ; et la préparation étant exécutée par précipitation sonochimique.

3. Composite antibactérien selon la revendication 1 destiné à l'utilisation en thérapie.

4. Composite antibactérien selon la revendication 1 destiné à l'utilisation en cosmétique.

5. Matériau comprenant le composite antibactérien selon la revendication 1, le matériau étant sélectionné dans le groupe constitué d'un filtre, d'un composant de masque facial, d'un respirateur, d'un climatiseur, d'un réfrigérateur, d'une machine à laver ou d'un aspirateur, d'un chewing-gum, d'un supplément diététique, d'une dispersion pour peinture des murs, d'une pâte dentifrice, d'un lavage buccal, d'un savon, d'un matériau de premier secours, d'un emplâtre adhésif, d'un bandage, d'une gaze, d'un ciment dentaire, d'un matériau de formation d'implant dentaire, d'un déodorant, d'un applicateur à bille, d'un shampooing, et d'un gel douche.
